# FASCICULE DE BREVET EUROPEEN

(11) **EP 2 976 043 B1**
(45) Date de publication et mention de la délivrance du brevet: **10.05.2017**
(21) Numéro de dépôt: 14716885.0
(22) Date de dépôt: 18.03.2014
(51) Int. Cl.: A61F 2/24, A61B 17/04, A61B 17/068, A61B 17/064

(54) **DISPOSITIF POUR REALISER UNE ANNULOPLASTIE PAR VOIE TRANSAPICALE DE LA VALVE MITRALE**
VORRICHTUNG ZUR DURCHFÜHRUNG EINER TRANSAPIKALEN MITRALKLAPPEN-ANNULOPLASTIE
DEVICE FOR CARRYING OUT A TRANSAPICAL MITRAL VALVE ANNULOPLASTY

(30) Priorité: 20.03.2013 FR 1352478
(43) Date de publication de la demande: 27.01.2016
(73) Titulaire: Le CHU de Saint-Etienne, 42055 Saint-Etienne Cedex 2 (FR); CMI'NOV, 43120 MONISTROL SUR LOIRE (FR)
(72) Inventeur: Vola, Marco, 42270 Saint-Proest-en Jarez (FR); Pain, Bernhard, 43120 Monistrol-sur-Loire (FR)
(74) Mandataire: Cabinet Laurent & Charras
(86) Numéro de dépôt international: PCT/FR2014/050627
(87) Numéro de publication internationale: WO 2014/147336

(56) Documents cités:
- WO-A2-2008/048626
- US-A1- 2011 106 247
- US-A1- 2012 245 604

## Description

L'invention concerne un dispositif pour réaliser une annuloplastie par voie transapicale de la valve mitrale.

. Autrement dit, l'annuloplastie a pour but de diminuer le calibre de l'anneau mitral en raccourcissant, par plicature de l'attache de la petite valve, le point d'appui étant pris entre les commissures. Par commissure, on entend rétrécissement du périmètre de la partie postérieure de l'anneau mitral en effectuant sur celui-ci des plicatures par des points, avec comme résultat, une diminution du diamètre antéro-postérieur et latéro -latéral de la valve mitrale

Une annuloplastie mitrale est effectuée en tant que moyen de correction d'une fuite mitrale dont le mécanisme est une dilatation de l'anneau mitral (avec perte de coaptation des berges valvulaires) ou, en complément de la correction de fuite avec un autre mécanisme, ( prolapsus de la valve mitrale) pour augmenter la coaptation de la valve mitrale postérieure par rapport à la valve mitrale antérieure

Une annuloplastie mitrale constitue une opération longue et lourde qui nécessite l'ouverture d'une des cavités cardiaques coeur et de la cage thoracique avec circulation sanguine extracorporelle.

Toutefois, de nombreuses solutions ont été proposées afin de réaliser des annuloplasties moins invasives, évitant à la fois un arrêt cardiaque pharmacologique (isquémique) du muscle cardiaque et la circulation extracorporelle. Parmi les dispositifs connus de traitement pour réaliser une annuloplastie, il est possible d'utiliser une navigation dans l'oreillette gauche soit par voie rétrograde, c'est-à-dire à partir de l'artère fémorale soit, par voie antérograde, c'est-à-dire par voie veineuse et transeptale. Toutefois, ces solutions rendent laborieux le repérage de l'anneau mitral. Il en résulte un investissement de temps relativement important.

Une solution avantageuse est d'utiliser la voie transapicale, c'est-à-dire de passer directement au niveau de l'apex du coeur.

Une solution de ce type ressort par exemple de l'enseignement du document WO2012/167095. Un autre document WO-A-2008/048626 divulgue un dispositif pour réaliser une annuloplastie par voie transapicale de la valve mitrale et destiné à être positionné dans un introducteur étanche disposé dans la cavité thoracique entre deux côtes pour pénétrer dans le ventricule gauche en passant par l'apex du coeur. Des difficultés peuvent également apparaître pour la fixation de l'implant prothétique au niveau de l'anneau mitral natif. Par exemple, la fixation de l'anneau mitral peut être effectuée soit par auto visage sur l'axe (US 2011/0106247) soit par solidarisation à une bande de tissu de l'anneau mitral natif au moyen de sutures sous forme de crochets.

Pour assurer la fixation en tant que telle, c'est-à-dire la liaison de l'implant prothétique au niveau de l'anneau mitral, les moyens actuellement mis en oeuvre permettent d'appliquer seulement une force unilatérale de sorte que l'on ne peut exclure des risques de fuites liées à une mauvaise fixation.

Avec ces systèmes, très souvent, plusieurs tentatives sont nécessaires pour obtenir une bonne fixation.

L'invention s'est fixée pour but de remédier à ces inconvénients de manière sûre, simple, efficace et rationnelle.

Le problème que se propose de résoudre l'invention est de faciliter la navigation du dispositif apte à réaliser l'annuloplastie le long de l'anneau mitral. Cette navigation s'effectue avec un simple mouvement de rétraction / rotation en utilisant la voie transapicale pour pénétrer dans le ventricule gauche en passant par l'apex du coeur.

Un autre objectif est de faciliter l'espacement des points de suture de l'implant par rapport à l'anneau mitral en réalisant une simple rotation sous échocardiographie 3D ou 2D ;

Un autre objectif est de pouvoir travailler sans circulation extra corporelle, ce qui nécessite un dispositif étanche au niveau du corps par un joint élastomère qui permet le passage des organes de commande des mouvements et empêche le sang de fuir.

Enfin, un autre but recherché est d'améliorer la fixation de l'implant par rapport à l'anneau mitral en exerçant une force d'appui et une force de contre-appui opposées pour effectuer une perforation avec précision et un passage d'un matériel d'ancrage dans un temps relativement court et sans fragiliser le tissu adjacent.

Pour résoudre un tel problème, il a été conçu et mis au point un dispositif pour réaliser une annuloplastie par voie transapicale de la valve mitrale et destiné à être positionné dans un introducteur étanche disposé dans la cavité thoracique entre deux côtes pour pénétrer dans le ventricule gauche en passant par l'apex du coeur. Selon l'intervention, le dispositif comprend un corps équipé d'une poignée et d'au moins un organe de commande apte à agir sur un ensemble pour la mise en place et la fixation d'une tresse au niveau de l'anneau mitral au moyen d'éléments de suture Le dit ensemble présente des moyens aptes à permettre l'extraction d'une suture au travers de l'anneau mitral natif en étant apte à enserrer la tresse et s'ancrer sur la périphérie de l'anneau mitral natif sous un effet de pincement de la dite suture en exerçant deux forces d'appui de pression opposées.

Pour résoudre le problème posé de la mise en place de la tresse au niveau de l'anneau mitral, l'ensemble est constitué par un bras tubulaire comprenant deux parties aptes à être séparées après introduction dans le ventricule gauche, l'une des parties reçoit les sutures et est apte à être mise au contact dans la commissure entre la valve mitrale et les parois du ventricule gauche, tandis que l'autre partie reçoit la tresse et est apte à passer à travers la valve mitrale et à être positionnée au droit de l'extrémité de la dite partie recevant les sutures.

Pour résoudre le problème posé d'assurer le positionnement dans l'espace de la tresse par rapport aux sutures, la partie du bras qui reçoit la tresse est réalisée en plusieurs éléments montés avec capacité de déplacement et d'orientation par rapport à la partie qui reçoit les sutures. L'extrémité libre de la partie qui reçoit la tresse est réalisée en deux éléments articulés. L'élément articulé de l'extrémité est apte à être positionné sensiblement perpendiculairement à l'extrémité de la partie qui reçoit les sutures.

Le problème posé de la mise en place des sutures en vue de la fixation de la tresse, qui constitue l'implant prothétique, par rapport à l'anneau mitral est résolu en ce que la partie du bras qui reçoit les sutures, présente à son extrémité une aiguille montée avec capacité de déplacement en translation pour déborder de ladite extrémité, ladite aiguille étant conformée pour permettre l'engagement, le guidage et le maintien de la suture en débordement de ladite extrémité.

En position de débordement de l'aiguille et de la suture et de débordement de la suture par rapport à l'aiguille, la dite suture débouche dans une ouverture que présente l'extrémité libre de la partie du bras qui reçoit la tresse afin de former, en considérant la nature du matériau constituant la suture, une boucle apte à enserrer une partie de la tresse disposée transversalement dans ladite ouverture de sorte qu'après retrait de l'aiguille, la suture n'est plus retenue pour s'ancrer sur la périphérie de l'anneau mitral.

Pour résoudre le problème posé de pouvoir enserrer la section de la tresse, cette dernière tresse logée dans deux canaux de guidage parallèles formés dans l'épaisseur de la partie correspondante du bras, de manière à former une boucle au niveau de l'ouverture de ladite partie, pour être enserré par la suture.

Pour résoudre le problème posé d'assurer le déploiement des deux éléments articulés d'extrémités de la partie qui reçoit la tresse, lesdits éléments sont accouplés, d'une manière articulée, à un élément mobile en translation assujetti à un organe de manoeuvre sous forme d'un curseur linéaire de sorte qu'un déplacement du curseur dans un sens provoque le déplacement linéaire et l'écartement de deux éléments d'extrémité par rapport à la partie du bras recevant les sutures en combinaison avec un agent dudit bras. L'agencement dudit bras est une rampe.

Le dispositif est également remarquable par les caractéristiques suivantes considérées en combinaison :
- l'élément d'extrémité de la partie du bras qui reçoit la tresse est assujetti à un câble relié à un organe de commande actionné par une gâchette que présente le corps de manière à rabaisser l'élément d'extrémité articulé en direction de l'extrémité de la partie du bras recevant les sutures.
- l'élément d'extrémité articulé du bras recevant la tresse est rappelé en position d'alignement avec l'autre élément articulé, par un organe élastique.
- l'élément d'extrémité articulé du bras recevant la tresse coopère en position rabattue avec une butée.
- les moyens aptes à permettre l'extraction d'une suture pour enserrer la tresse et s'ancrer sur la périphérie de l'anneau mitral natif sont constitués par une came profilée pour distribuer les sutures, une came profilée pour l'implantation des sutures et une came pour le déplacement linéaire de l'aiguille, lesdites cames étant assujetties à un système de pignons asservis à la gâchette de commande et à un système de câbles reliés à des organes poussoirs.

Un autre problème que se propose de résoudre l'invention est de permettre, après mise en place et fixation de la tresse constituant un implant prothétique par rapport à l'anneau mitral, de réduire le serrage de l'anneau de la valve pour éviter tout risque de fuite.

Dans ce but, la tresse est assujettie à des moyens aptes à permettre de réduire sa circonférence, après mise en place et fixation sur la périphérie de l'anneau mitral. Les moyens sont constitués par un cordon de traction monté librement en translation dans l'âme centrale de la tresse.

L'invention est exposée ci-après plus en détail à l'aide des figures des dessins annexé dans lesquels :
La figure 1 est une vue en coupe à caractère schématique montrant le principe d'utilisation du dispositif pour réaliser une annuloplastie selon l'invention,
La figure 2 montre un type d'ancrage obtenu,
La figure 3 est une vue partielle en coupe à caractère schématique montrant le principe de mise en place d'une suture par rapport à la tresse constituant l'implant,
La figure 4 montre une annuloplastie réalisée au moyen du dispositif selon l'invention,
La figure 5 est une vue en perspective de l'annuloplastie correspondant à la figure 4,

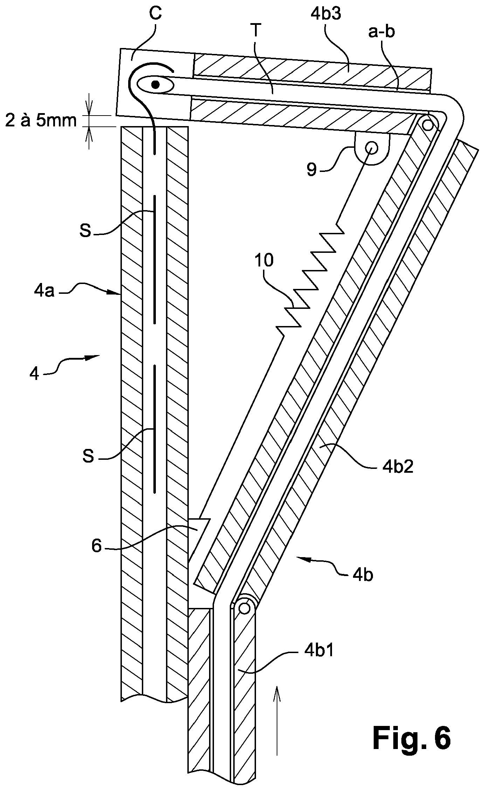

La figure 6 est une vue en coupe et à caractère schématique montrant les extrémités des bras de l'ensemble, pour la mise en place des sutures métalliques en relation avec la tresse,

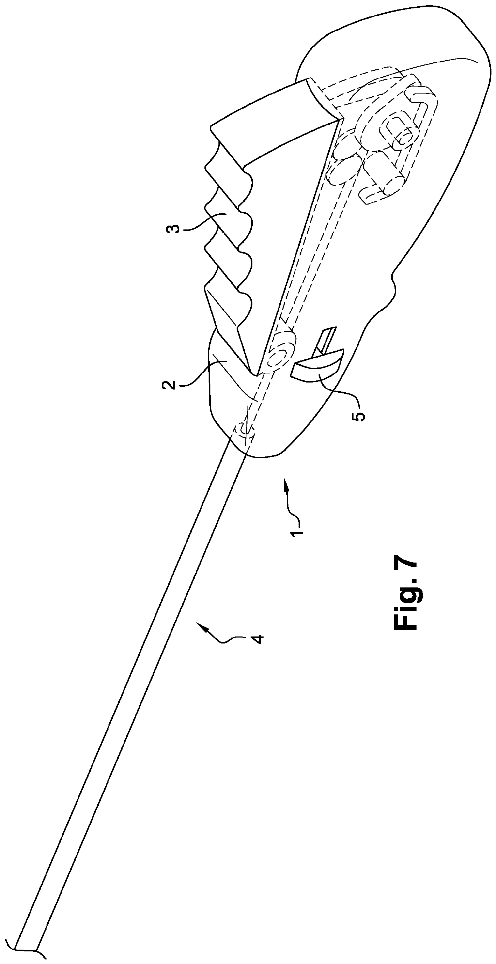

La figure 7 est une vue en perspective d'une forme de réalisation du dispositif,

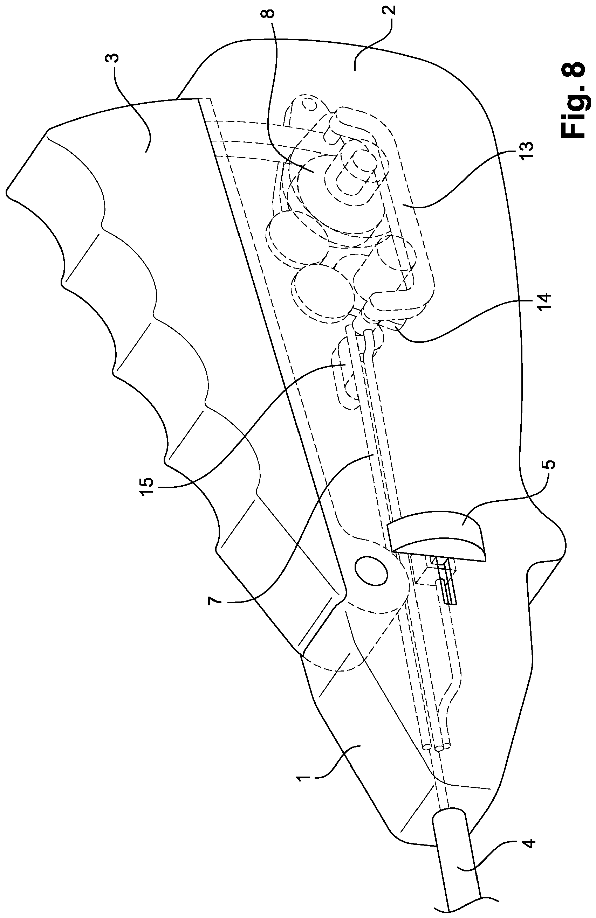

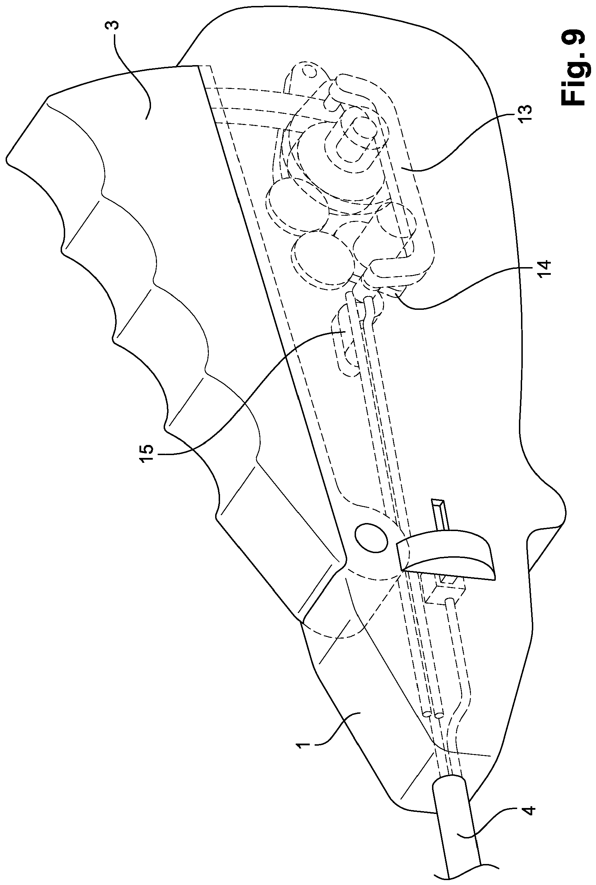

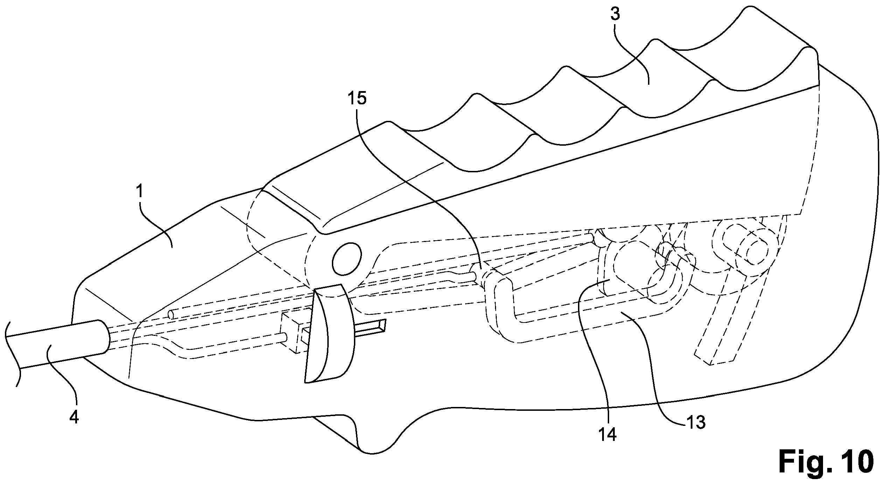

Les figures 8, 9 et 10 sont des vues en perspective du dispositif au niveau du corps de la poignée de commande montrant le mécanisme de commande selon différentes positions en vue de la commande des éléments articulés composants le bras en fonction du positionnement d'un curseur (figures 8 et 9) et après action sur la poignée de manoeuvre (figure 10)
Les figures 11, 12, 13, 14 et 15 sont des vues en perspective de l'extrémité du bras selon différentes positions, à savoir :

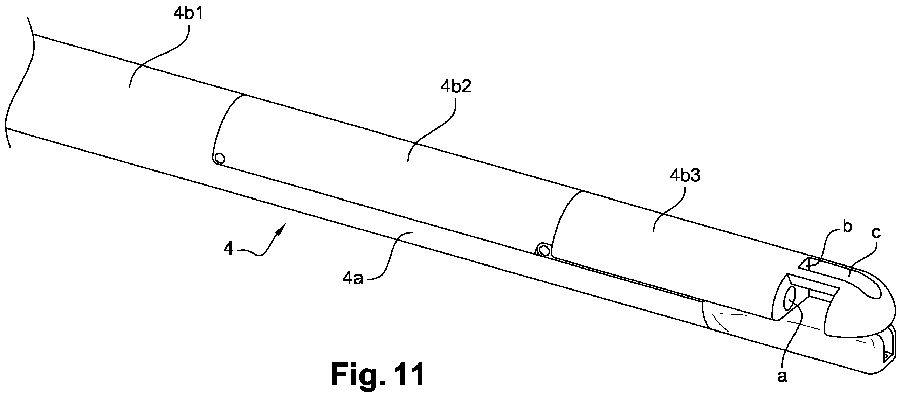

   - figure 11 : alignement des différents éléments de l'extrémité du bras correspondant à l'introduction du dispositif.

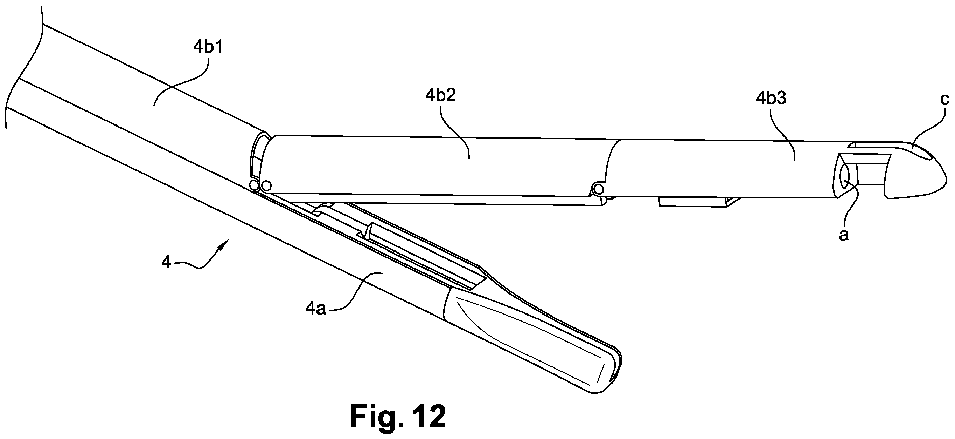

   - figure 12 : Ouverture du bras après introduction.

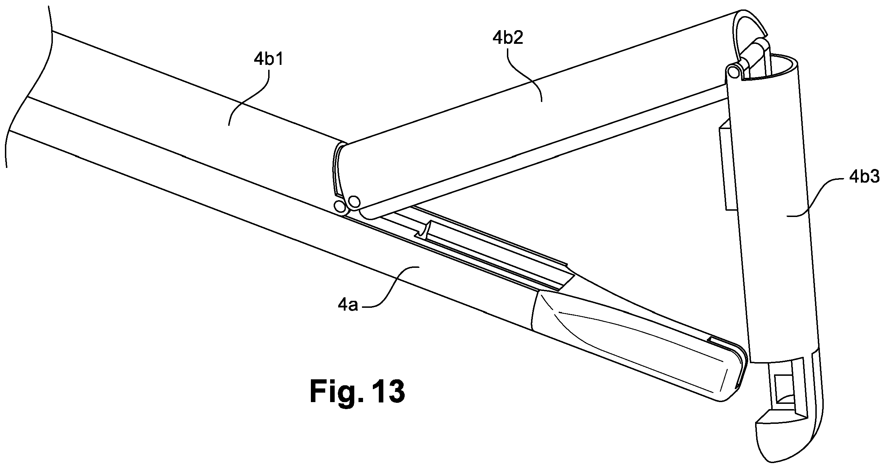

   - figure 13 : Commande de l'élément d'extrémité en position de rabattement au niveau d'une partie du bras recevant la tresse.

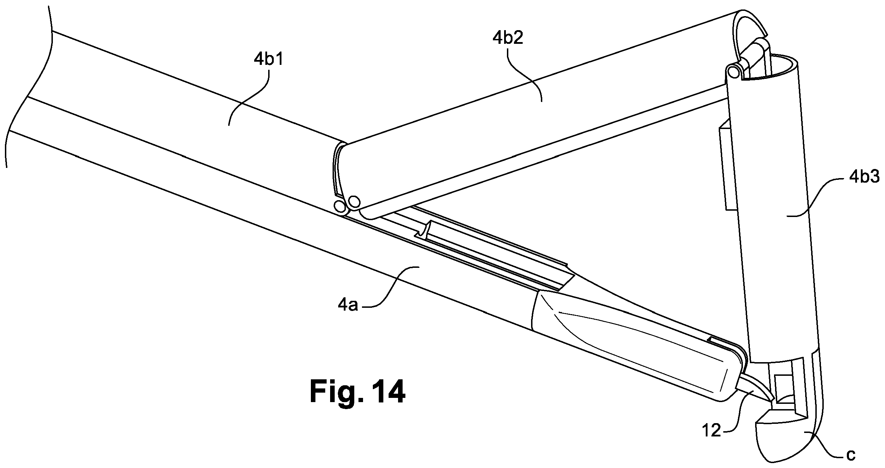

   - figure 14 : Commande de l'aiguille.

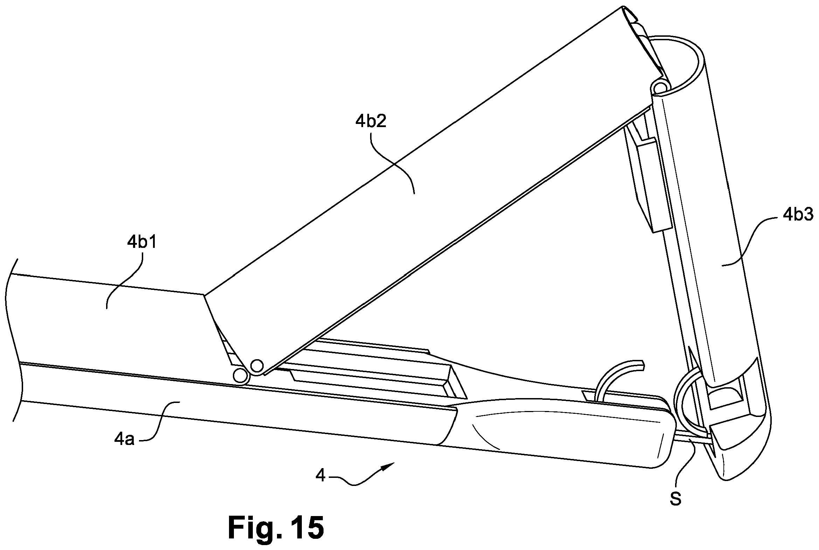

   - figure 15 : Réalisation de la boucle.

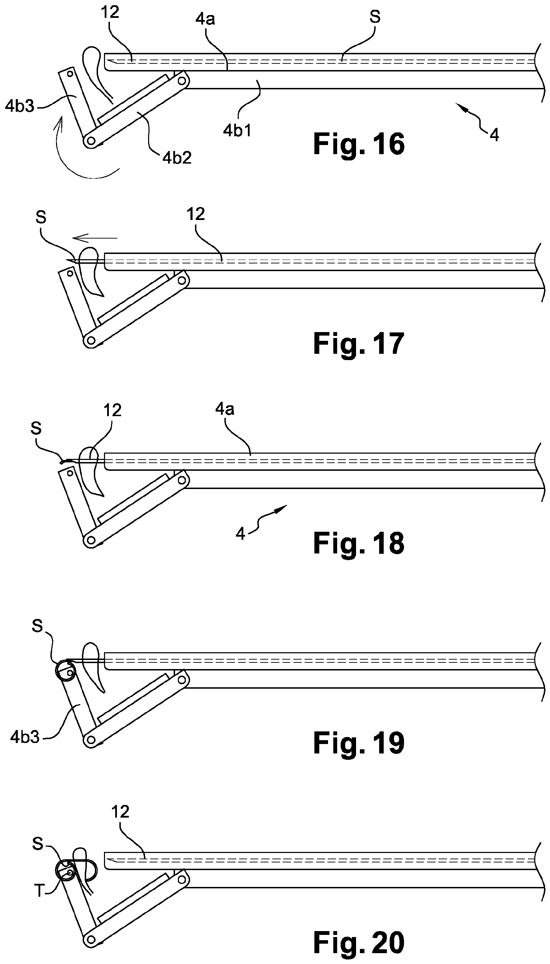

   - les figures 16 à 20 montrent le principe de réalisation de la boucle par rapport à la suture.

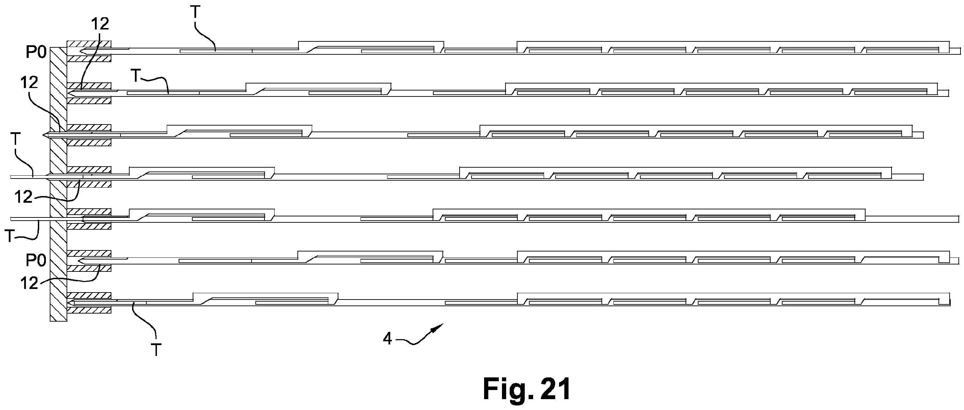

   - La figure 21 : est une cinématique de présentation des sutures.

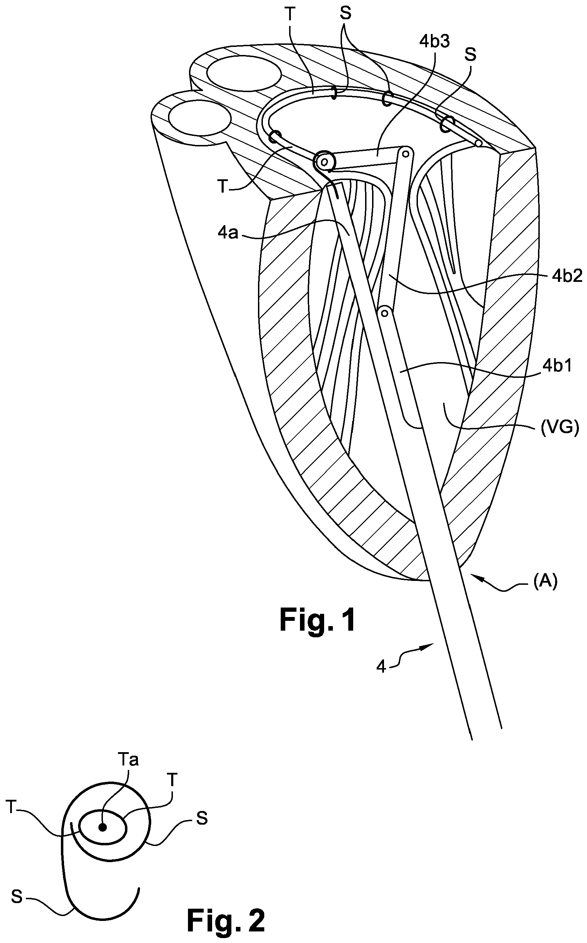

Le dispositif est destiné à être positionné dans un introducteur étanche de tout type connu et approprié (non représenté) disposé dans la cavité thoracique entre deux côtes pour pénétrer dans le ventricule gauche (VG) en passant par l'apex (A) du coeur (figure 1).

Le dispositif comprend un corps (1) équipé d'une poignée (2) et d'au moins un organe de commande (3), sous forme par exemple d'une gâchette, pour agir sur un ensemble en vue de la mise en place et de la fixation d'un implant prothétique sous forme d'une tresse (T) au niveau de l'anneau mitral, au moyen d'éléments de suture (S).

L'ensemble est constitué par un bras tubulaire (4) comprenant deux parties (4a) et (4b) aptes à être écartées après introduction dans le ventricule gauche (VG). L'une des parties (4a) reçoit les sutures (S) et est destinée à être mise au contact dans la commissure entre la valve mitrale et les parois du ventricule gauche. L'autre partie (4b) reçoit la tresse (T) et est conformée pour être engagée au travers la valve mitrale afin d'être positionnée au droit de l'extrémité de la partie (4a) recevant les sutures comme il sera indiqué dans la suite de la description.

La partie du bras (4b) qui reçoit la tresse est réalisée en plusieurs éléments (4b1), (4b2), (4b3) montés avec capacité de déplacement et d'orientation par rapport à la partie (4a) du bras (4).

Les deux éléments (4b2) et (4b3) sont articulés, l'élément articulé d'extrémité (4b3) étant apte à être positionné sensiblement perpendiculairement à l'extrémité de la partie (4a) qui reçoit les sutures (S). Ces deux éléments articulés d'extrémité (4b2) et (4b3) sont également accouplés de manière articulée, à un élément (4b1) qui constitue un charriot mobile en translation, assujetti à un organe de manoeuvre sous forme d'un curseur (5).

Un déplacement linéaire du curseur (5) dans un sens, provoque le déplacement linéaire du charriot mobile (4b1) et l'écartement des deux éléments d'extrémité (4b2) et (4b3) par rapport à la partie du bras (4a) en combinaison avec, une rampe (6), par exemple. Ainsi la partie du bras (4b) passe dans la position illustrée figure 11 à la position illustrée figure 12.

Suivant une autre caractéristique, l'élément d'extrémité (4b) est assujetti à un câble (7) lié à un organe de commande sous forme, par exemple, d'une came (8) actionné par la gâchette (3) de manière à rabaisser ledit élément d'extrémité (4b3) en direction de l'extrémité de la partie du bras (4a) recevant les sutures. Dans cette position rabattue de l'élément de l'extrémité (4b2), ce dernier coopère avec un élément (9) faisant office de butée. Un ressort (10) assure le rappel en position d'alignement des éléments (4b2) et (4b3).

Tous les organes de commande passent au travers d'un joint qui assure l'étanchéité vis-à-vis du sang entre la poignée et l'intérieur de la cavité cardiaque.

La tresse (T) est logée avec capacité de coulissement dans deux canaux de guidage parallèles (a) et (b) formés dans l'épaisseur de la partie (4b) du bras (4). Ces deux canaux (a) et (b) débouchent dans une ouverture (c) formée à l'extrémité libre de l'élément (4b2) du bras pour permettre à la tresse (T) de former une boucle (TA) apte à être enserrée par une suture comme il sera indiqué dans la suite de la description.

Selon une autre caractéristique, l'extrémité libre (4a) du bras (4) présente une aiguille (12) montée avec capacité de déplacement en translation pour déborder de ladite extrémité. Cette aiguille (12) est conformée pour permettre l'engagement, le guidage et le maintien de la suture (S) en débordement de ladite extrémité. On rappelle à cet égard, de manière connue, que les différentes sutures sont réalisées dans un matériau à mémoire de forme de sorte qu'après avoir été libérée de ladite aiguille, la suture va automatiquement être déformée pour créer une boucle d'ancrage.

Dans cette position de débordement de l'aiguille (12) recevant la suture (figure 17) et de débordement de la suture par rapport à l'aiguille (figure 18), la suture (S) débouche dans l'ouverture (c) formée, comme indiqué précédemment, à l'extrémité libre de l'élément (4b2) où les deux brins de la suture forment la boucle. Après retrait de l'aiguille (12) (figure 14), en considérant la nature du matériau constituant la suture (S), cette dernière forme automatiquement une boucle d'ancrage apte à enserrer la partie de la tresse disposée transversalement dans ladite ouverture (c) après s'être ancrée au travers du tissu de l'anneau mitral. Dans ce but, pour permettre l'extraction d'une suture (S), le corps de l'appareil présente une came profilée (13) pour distribuer les sutures, une came profilée (14) pour l'implantation des sutures et une came (15) pour le déplacement linéaire de l'aiguille (12). Lesdites cames sont assujetties à un système de pignon (17) asservi à la gâchette de commande (3) et sont reliées à un système de câbles pour agir sur des moyens aptes à permettre, comme indiqué, de distribuer et d'implanter les sutures en combinaison avec le déplacement de l'aiguille.

En référence à la figure 1 des dessins, l'utilisation du dispositif selon l'invention peut être exposée comme suit :
Le bras (4) est disposé dans un introducteur, par exemple, d'un diamètre inférieur ou égal à 8 mm disposé dans la cavité thoracique, entre deux côtes avec, pour objectif, de le faire pénétrer dans le ventricule gauche (VG) en passant par l'apex (A) du coeur. De manière connue, cet introducteur a pour objectif de permettre le passage du dispositif nécessaire à l'intervention, de façon totalement étanche. Le bras (4) est ensuite introduit, sous contrôle radiographique, dans l'introducteur ainsi mis en place. Après introduction dans le ventricule gauche, les deux parties (4a) et (4b) sont écartées après avoir agi sur le curseur (4).

La partie (4a) du bras (4) qui reçoit les sutures métalliques à mémoire de forme (S) est positionnée au contact de la commissure entre la valve mitrale et les parois du ventricule gauche (VG).

La partie (4b) qui reçoit la tresse (T) et qui est composée des différents éléments articulés (4b1), (4b2), (4b3), passe à travers la valve mitrale dans la position ouverte et en alignement des segments (4b2) et (4b3) (figure 12).

On appuie ensuite sur la gâchette (3) pour permettre, comme indiqué précédemment, de rabattre l'élément d'extrémité (4b3) très sensiblement au droit de l'extrémité de la partie (4a) du bras (4). De manière concomitante on provoque l'extraction d'une suture.

On renvoie aux figures 16 à 20 et à la cinématique de la figure 21 qui montre la présentation des sutures et la réalisation de la boucle d'ancrage. Sous l'effet de l'action de la came de distribution une suture est engagée dans l'aiguille (12), laquelle est ensuite déplacée linéairement sous l'effet de la came pour traverser le tissu de l'anneau mitral. La suture est ensuite poussée en dehors de l'aiguille (12) pour être positionnée au droit de l'ouverture (c) que présente l'extrémité libre de l'élément (4b3), c'est-à-dire au droit de la partie de la suture qui forme une boucle (TA). Après extraction de l'aiguille (12), la suture est totalement libérée afin de constituer, de manière concomitante, sous l'effet de la mémoire de forme du matériau la constituant, une boucle d'ancrage à la périphérie de l'anneau (figures 2, 4 et5).

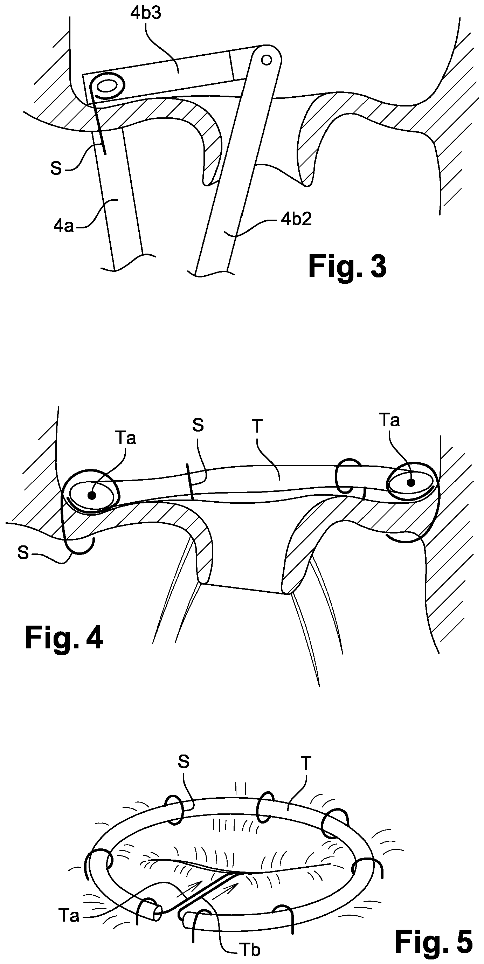

Le chirurgien répète l'opération plusieurs fois en effectuant un simple mouvement de rétraction/rotation sous contrôle radiographique afin de positionner le nombre de sutures nécessaires sur la périphérie de l'anneau de la valve (figure 5).

Suivant une autre caractéristique importante, la tresse (T) apte à constituer l'implant prothétique et qui peut être réalisée en polyester ou tout autre matériau parfaitement connu pour ce type d'application, est assujettie à un moyen apte à permettre de réduire sa circonférence après sa mise en place et fixation sur la périphérie de l'anneau mitral, comme indiqué. Par exemple, ces moyens sont constitués par un cordon de traction (F) monté librement en translation et à libre coulissement dans l'âme centrale de la tresse (T) pour permettre, sous un effet de traction, d'assurer le fronçage de la tresse et, par conséquent, la diminution de son diamètre. Ces dispositions sont particulièrement importantes pour permettre après la fixation de la tresse dans les conditions indiquées, de parfaitement adapter le diamètre de l'implant en s'assurant qu'il n'y a plus aucune fuite.

Dans ce but, le chirurgien retire le dispositif, et seules dépassent de l'introducteur, les deux extrémités du fil (F) qui constitue l'âme axiale de la tresse. Sous contrôle radio, en exerçant une simple fraction sur le fil, on provoque de manière concomitante le serrage de l'anneau de la valve. Après avoir trouvé la bonne tension correspondant au bon diamètre, le fil peut être serti au moyen d'un noeud ou d'un clip, puis coupé.

Les caractéristiques du dispositif selon l'invention apportent de nombreux avantages par rapport aux solutions existantes. Le fait de passer par la voie transapicale à partir de l'apex, permet à l'opérateur de facilement diriger le dispositif pour changer l'angulation et la direction de son emplacement ou de son mouvement de recul. Avec une coupe échographique bidimensionnelle de la jonction auriculo ventriculaire, il est possible de visualiser l'enclavement souhaité du dispositif sur la petite valve mitrale du côté ventriculaire. La mise en place est guidée par l'anatomie même des tissus et de la résistance que l'opérateur perçoit en position de butée de l'extrémité du bras (4). A cet égard, on observe que l'extrémité du bras est biseautée et arrondie pour pouvoir naviguer en passant entre les cordages de l'appareil mitral et s'enclaver dans l'angle dièdre au fond duquel sont injectées les différentes sutures. On souligne également la facilité de navigation du dispositif avec un simple mouvement de rétraction/rotation par la voie transapicale ventriculaire gauche pour la mise en place des différentes sutures d'ancrage.

A noter également que le dispositif permet d'exercer deux forces d'appui et de contre-appui opposées pour effectuer la perforation des tissus et le passage des sutures d'ancrage sans risque de fragiliser ledit tissu en supprimant tout risque de tâtonnement. Autrement dit, le tissu de l'anneau mitral et les tissus adjacents sont perforés plus efficacement en une seule fois avec une tenue optimale des agrafes de suture avec, à chaque fois, un contrôle échographique en 2D ou en 3D des phases du cycle d'agrafage.

## Revendications

1. Dispositif pour réaliser une annuloplastie par voie transapicale de la valve mitrale et destiné à être positionné dans un introducteur étanche disposé dans la cavité thoracique entre deux côtes pour pénétrer dans le ventricule gauche en passant par l'apex du coeur pour lequel il comprend un corps (1) équipé d'une poignée (2) et d'au moins un organe de commande (3) apte à agir sur un ensemble pour la mise en place et la fixation d'une tresse (T) au niveau de l'anneau mitral au moyen d'éléments de suture (S), **caractérisé en ce que** ledit ensemble présente des moyens aptes à permettre l'extraction d'une suture au travers de l'anneau mitral en étant apte à enserrer la tresse et s'ancrer sur la périphérie de l'anneau mitral sous un effet de pincement de ladite suture en exerçant deux forces d'appui de pression opposées.

2. Dispositif selon la revendication 1 ***caractérisé* en ce que** l'ensemble est constitué par un bras tubulaire (4) comprenant deux parties (4a) et (ab) aptes à être séparées après introduction dans le ventricule gauche, l'une des parties (4a) reçoit les sutures (S) et est apte à être mise au contact dans la commissure entre la valve mitrale et les parois du ventricule gauche, tandis que l'autre partie (4b) reçoit la tresse (T) et est apte à passer à travers la valve mitrale et à être positionnée au droit de l'extrémité de ladite partie recevant les sutures.

3. Dispositif selon la revendication 2 ***caractérisé* en ce que** la partie (4b) du bras qui reçoit la tresse (T) est réalisée en plusieurs éléments (4a), (4b), (4c) montés avec capacité de déplacement et d'orientation par rapport à la partie (4a) qui reçoit les sutures.

4. Dispositif selon la revendication 3 ***caractérisé* en ce que** l'extrémité libre de la partie (4b) qui reçoit la tresse est réalisée en deux éléments articulés (4b2) et (4b3), l'élément articulé d'extrémité (4b3) est apte à être positionné sensiblement perpendiculairement à l'extrémité de la partie (4a) qui reçoit les sutures.

5. Dispositif selon la revendication 2 ***caractérisé* en ce que** la partie (4a) du bras qui reçoit les sutures, présente, à son extrémité, une aiguille (12) montée avec capacité de déplacement en translation pour déborder de ladite extrémité, ladite aiguille (12) étant conformée pour permettre l'engagement, le guidage et le maintien de la suture (S) en débordement de ladite extrémité.

6. Dispositif selon la revendication 5 ***caractérisé* en ce qu'**en position de débordement de l'aiguille (12) et de la suture (S) et de débordement de la suture par rapport à l'aiguille (12), la dite suture (S) débouche dans une ouverture (c) que présente l'extrémité libre de la partie du bras qui reçoit la tresse afin de former, en considérant la nature du matériau constituant la suture, une boucle apte à enserrer une partie de la tresse disposée transversalement dans ladite ouverture (c) après retrait de l'aiguille (12), la suture n'étant plus retenue pour s'ancrer sur la périphérie de l'anneau mitral.

7. Dispositif selon la revendication 6 ***caractérisé* en ce que** la tresse (T) est logée dans deux canaux de guidage parallèles formés dans l'épaisseur de la partie correspondante (4b) du bras, de manière à former une boucle au niveau de l'ouverture (c) de ladite partie pour être enserrée par la suture.

8. Dispositif selon l'une quelconque des revendications 2, 3 et 4 ***caractérisé* en ce que** les deux éléments articulés d'extrémité (4b2) et (4b3) de la partie qui reçoit la tresse sont accouplés d'une manière articulée à un élément mobile en translation (4b) assujetti à un organe de manoeuvre sous forme d'un curseur linéaire de sorte qu'un déplacement du curseur dans un sens provoque le déplacement linéaire et l'écartement de deux éléments d'extrémité par rapport à la partie du bras recevant les sutures en combinaison avec un agencement dudit bras.

9. Dispositif selon la revendication 8 ***caractérisé* en ce que** l'agencement dudit bras est une rampe (6).

10. Dispositif selon l'une quelconque des revendications 2, 3 et 4 ***caractérisé* en ce que** l'élément d'extrémité (4b3) de la partie du bras (4) qui reçoit la tresse (T) est assujetti à un câble relié à un organe de transmission actionné par l'organe de commande que présente le corps de manière à rabaisser l'élément d'extrémité articulé en direction de l'extrémité de la partie (4a) du bras recevant les sutures.

11. Dispositif selon l'une quelconque des revendications 2, 3, 4 et 10 ***caractérisé* en ce que** l'élément d'extrémité articulé (ab3) du bras (4) recevant la tresse (T) est rappelé en position d'alignement avec l'autre élément articulé par un organe élastique (10).

12. Dispositif selon la revendication 11 ***caractérisé* en ce que** l'élément d'extrémité articulé (4b3) du bras recevant la tresse coopère en position rabattue avec une butée (9).

13. Dispositif selon l'une quelconque des revendications 1 à 12 ***caractérisé* en ce que** les moyens aptes à permettre l'extraction d'une suture pour enserrer la tresse et s'ancrer sur la périphérie de l'anneau mitral sont constitués par une came profilée pour distribuer les sutures, une came profilée pour l'implantation des sutures et une came pour le déplacement linéaire de l'aiguille, lesdites cames étant assujetties à un système de pignons asservis à la gâchette de commande et à un système de câbles.

14. Dispositif selon la revendication 1 ***caractérisé* en ce que** la tresse (T) est assujettie à des moyens aptes à permettre de réduire sa circonférence, après mise en place et fixation sur la périphérie de l'anneau mitral.

15. Dispositif selon la revendication 14 ***caractérisé* en ce que** les moyens sont constitués par un cordon de traction monté librement en translation dans l'âme centrale de la tresse.

## Patentansprüche

1. Vorrichtung zur Durchführung einer Anuloplastie der Mitralklappe durch transapikalen Zugang und zum Platzieren in eine dichte Einführungsvorrichtung, die in der Brusthöhle zwischen zwei Rippen zum Einführen in den linken Ventrikel über die Herzspitze angeordnet ist, bestehend aus einem Gehäuse (1) mit einem Griffstück (2) und mindestens einem Bedienorgan (3), das auf eine Einheit zur Anbringung und Befestigung eines Geflechts (T) am Mitralring mithilfe von Nahtmaterial (S) einwirken kann, **dadurch gekennzeichnet, dass** diese Einheit Mittel aufweist, die eine Extraktion von Nahtmaterial durch den Mitralring zulässt, wobei die Einheit das Geflecht anbinden und an der Außenkontur des Mitralrings durch Einschnürwirkung des Nahtmaterials verankern kann, indem zwei entgegengesetzt gerichtete Auflagedruckkräfte ausgeübt werden.

2. Vorrichtung nach Anspruch 1, **dadurch gekennzeichnet, dass** die Einheit aus einer rohrförmigen Armkonstruktion (4) bestehend aus zwei Teilen (4a) und (4b) gebildet wird, die nach Einführung in den linken Ventrikel trennbar sind, wobei ein Teil (4a) das Nahtmaterial (S) enthält und in der Kommissur zwischen Mitralklappe und Wänden des linken Ventrikels in Kontakt gebracht werden kann, während der andere Teil (4b) das Geflecht (T) enthält und durch die Mitralklappe führen und auf gleicher Höhe wie das Ende des Teils, der das Nahtmaterial erhält, positioniert werden kann.

3. Vorrichtung nach Anspruch 2, **dadurch gekennzeichnet, dass** der Teil (4b) der Armkonstruktion, der das Geflecht (T) enthält, aus mehreren Elementen (4a), (4b), (4c) besteht, welche, bezogen auf den Teil (4a), der das Nahtmaterial enthält, verschiebbar und ausrichtbar montiert sind.

4. Vorrichtung nach Anspruch 3, **dadurch gekennzeichnet, dass** das freie Ende des Teils (4b), der das Geflecht enthält, aus zwei Gelenkstücken (4b2) und (4b3) gebildet ist, wobei das Gelenkendstück (4b3) im wesentlichen senkrecht zum Ende des Teils (4a), der das Nahtmaterial enthält, positioniert werden kann.

5. Vorrichtung nach Anspruch 2, **dadurch gekennzeichnet, dass** der Teil (4a) der Armkonstruktion, der das Nahtmaterial enthält, an seinem Ende eine Nadel (12) aufweist, die zum Überstehen über dieses Ende hinaus in Translationsrichtung verschiebbar montiert ist, wobei diese Nadel (12) derart angepasst ist, dass sie das Einsetzen, das Führen und das Halten des Nahtmaterials (S) im Überstand dieses Endes ermöglicht.

6. Vorrichtung nach Anspruch 5, **dadurch gekennzeichnet, dass** in Überstandsposition der Nadel (12) und des Nahtmaterials (S) und in Überstandsposition des Nahtmaterials bezogen auf die Nadel (12) das Nahtmaterial (S) in eine Öffnung (c) mündet, die am freien Ende des Teils der Armkonstruktion ausgebildet ist, der das Geflecht enthält, um unter Berücksichtigung der Nahtmaterialbeschaffenheit eine Schlinge zu bilden, die nach Rückzug der Nadel (12) einen Teil des transversal in der Öffnung (c) angeordneten Geflechts anbinden kann, wobei das Nahtmaterial zum Verankern an der Außenkontur des Mitralrings nicht mehr verwendet wird.

7. Vorrichtung nach Anspruch 6, **dadurch gekennzeichnet, dass** das Geflecht (T) in zwei parallelen Führungskanälen untergebracht ist, die in der Dicke des entsprechenden Teils (4b) der Armkonstruktion ausgebildet sind, so dass im Bereich der Öffnung (c) dieses Teils eine Schlinge gebildet wird, um durch das Nahtmaterial angebunden zu werden.

8. Vorrichtung nach einem der Ansprüche 2, 3 und 4, **dadurch gekennzeichnet, dass** die beiden Gelenkendstücke (4b2) und (4b3) des Teils, der das Geflecht enthält, gelenkig an ein in Translationsrichtung bewegliches Element (4b) gekoppelt sind, welches an ein Bedienorgan in Form eines linear bewegbaren Schiebers gebunden ist, sodass eine Bewegung des Schiebers in eine Richtung eine lineare Bewegung und ein Aufweiten der beiden Endstücke bezogen auf den Teil der Armkonstruktion, der das Nahtmaterial enthält, zusammen mit einer Ausrichtung der Armkonstruktion bewirkt.

9. Verfahren nach Anspruch 8, **dadurch gekennzeichnet, dass** es sich bei der Ausrichtung der Armkonstruktion um einen Ausleger (6) handelt.

10. Vorrichtung nach einem der Ansprüche 2, 3 und 4, **dadurch gekennzeichnet, dass** das Endstück (4b3) des Teils der Armkonstruktion (4), der das Geflecht (T) enthält, an ein mit einem Übertragungsorgan verbundenen Seil gebunden ist, welches über das das Gehäuse darstellende Bedienorgan betätigt wird, um das Gelenkendstück in Richtung des Ende des Teils (4a) der Armkonstruktion abzusenken, der das Nahtmaterial enthält.

11. Vorrichtung nach einem der Ansprüche 2, 3, 4 und 10, **dadurch gekennzeichnet**, das Gelenkendstück (4b3) der Armkonstruktion (4), das das Geflecht (T) enthält, über ein federndes Element (10) bezogen auf das andere Gelenkstück in Ausrichtposition zurückgeholt wird.

12. Vorrichtung nach Anspruch 11, **dadurch gekennzeichnet, dass** das Gelenkendstück (4b3) der Armkonstruktion, das das Geflecht enthält, in eingeklappter Position mit einem Anschlag (9) zusammenwirkt.

13. Vorrichtung nach einem der Ansprüche 1 bis 12, **dadurch gekennzeichnet, dass** die Mittel zur Extraktion von Nahtmaterial zum Anbinden des Geflechts und zum Verankern an der Außenkontur des Mitralrings aus einer profilierten Nocke zur Bereitstellung des Nahtmaterials, einer profilierten Nocke zur Implantation des Nahtmaterials und einer Nocke zur linearen Bewegung der Nadel gebildet sind, wobei die Nocken an ein Ritzelsystem gebunden sind und die Ritzel über einen Drücker und ein Seilsystem gesteuert werden.

14. Vorrichtung nach Anspruch 1, **dadurch gekennzeichnet, dass** das Geflecht (T) an Mittel gebunden ist, die den Geflechtumfang nach Anbringung und Befestigung des Mitralrings an der Außenkontur reduzieren können.

15. Vorrichtung nach Anspruch 14, **dadurch gekennzeichnet, dass** die Mittel als in Translationsrichtung lose Zugschnur in der zentralen Seele des Geflechts ausgebildet sind.

## Claims

1. A device for performing a transapical mitral valve annuloplasty and designed to be positioned in a sealed inserter placed in the thoracic cavity between two ribs so as to penetrate into the left ventricle by passing through the apex of the heart, for which it comprises a body (1) equipped with a handle (2) and with at least one control member (3) suitable for acting on an assembly for putting in place and for fastening a braid (T) to the mitral annulus by means of suture elements (S), **characterized in that** said assembly has means suitable for enabling a suture to be extracted through the mitral annulus for clamping the braid and for anchoring to the periphery of the mitral annulus under a pinching effect of said suture exerting two mutually opposing pressing forces.

2. Device according to claim 1, **characterized in that** the assembly is constituted by a tubular arm (4) comprising two portions (4a) and (4b) that are suitable for being separated after being inserted into the left ventricle, one of the portions (4a) receives the sutures (S) and is suitable for being put into contact in the commissure between the mitral valve and the walls of the left ventricle, while the other portion (4b) receives the braid (T) and is suitable for passing through the mitral valve and for being positioned in line with the end of said portion receiving the sutures.

3. Device according to claim 2, **characterized in that** the portion (4b) of the arm that receives the braid (T) is made up several elements (4a), (4b), 4c), mounted with displacement and orientation capability relative to the portion (4a) that receives the sutures.

4. Device according to claim 3, **characterized in that** the free end of the portion (4b) that receives the braid is made up of two hinged elements (4b2) and (4b3), the end hinged element (4b3) is suitable for being positioned substantially perpendicularly to the end of the portion (4a) that receives the sutures.

5. Device according to claim 2, **characterized in that** the portion (4a) of the arm that receives the sutures has, at its end, a needle (12) mounted with translation capability so as to protrude beyond said end, said needle (12) being shaped to enable the suture (S) to be engaged, guided, and held protruding beyond said end.

6. Device according to claim 5, **characterized in that**, when the needle (12) and the suture (S) are in the protruding position, and when the suture is protruding relative to the needle (12), said suture (S) leads into an opening (c) in the free end of the portion of the arm that receives the braid in order to form, considering the nature of the material of the suture, a loop that is suitable for clamping a portion of the braid disposed transversely in said opening (c) after the needle (12) has been removed, the suture no longer being restrained so that it anchors onto the periphery of the mitral annulus.

7. Device according to claim 6, **characterized in that** the braid (T) is received in two parallel guide channels formed in the thickness of the corresponding portion (4b) of the arm, such as to form a loop at the opening (c) of said portion so as to be clamped by the suture.

8. Device according to any one of claims 2, 3, and 4, **characterized in that** the two end hinged elements (4b2) and (4b3) of the portion that receives the braid are coupled in hinged manner to an element (4b) movable in translation and secured to an operating member in the form of a linear slide so that a slide moving in one direction causes the two end elements to move linearly and to move away from the portion of the arm that receives the sutures in combination with an arrangement of said arm.

9. Device according to claim 8, **characterized in that** the arrangement of said arm is a ramp (6).

10. Device according to any one of claims 2, 3, and 4, **characterized in that** the end element (4b3) of the portion of the arm (4) that receives the braid (T) is secured to a cable connected to a transmission member actuated by the control member with which the body is provided in such a manner as to lower the end hinged element towards the end of the portion (4a) of the arm receiving the sutures.

11. Device according to any one of claims 2, 3, 4, and 10, **characterized in that** the end hinged element (4b3) of the arm (4) receiving the braid (T) is urged back into a position aligned with the other hinged element by a return resilient member (10).

12. Device according to claim 11, **characterized in that** the end hinged element (4b3) of the arm receiving the braid co-operates with an abutment (9) when it is in the folded-down position.

13. Device according to any one of claims 1 to 12, **characterized in that** the means suitable for enabling a suture to be extracted for clamping the braid and for anchoring onto the periphery of the mitral annulus are constituted by a shaped-section cam for dispensing the sutures, by a shaped-section cam for implanting the sutures and by a cam for linearly moving the needle, said cams being secured to a gear system enslaved to the control trigger and to a cable system.

14. Device according to claim 1, **characterized in that** the braid (T) is secured to means suitable for enable to reduce its circumference, after it has been put in place and fastened to the periphery of the mitral annulus.

15. Device according to claim 14, **characterized in that** the means are constituted by a pull cord mounted to be free to move in translation in the central core of the braid.
